# EUROPEAN PATENT SPECIFICATION

(11) **EP 3 484 277 B1**
(45) Date of publication and mention of the grant of the patent: **30.06.2021**
(21) Application number: 17740166.8
(22) Date of filing: 27.06.2017
(51) Int. Cl.: A01H 1/08, A01H 4/00, C12Q 1/68, A01H 1/04

(54) **DETERMINING THE GENOTYPE OF A MALE GAMETIC CELL**
BESTIMMUNG DES GENOTYPS EINER MÄNNLICHEN GAMETENZELLE
DÉTERMINATON DU GÉNOTYPE D'UNE CELLULE GAMÉTIQUE MÂLE

(30) Priority: 15.07.2016 US 201615211053
(43) Date of publication of application: 22.05.2019
(73) Proprietor: Pioneer Hi-Bred International, Inc., Johnston, Iowa 50131-1014 (US)
(72) Inventor: CRISMANI, Wayne Matthew, Fitzroy, Victoria 3065 (AU); DAY, Kevin, Johnston, Iowa 50131-0552 (US); MAY, Gregory D., Johnston, Iowa 50131-0552 (US); SCHARES, Justin Andrew, Johnston, Iowa 50131-0552 (US); YUN, Yue, Johnston, Iowa 50131-0552 (US)
(74) Representative: J A Kemp LLP
(86) International application number: PCT/US2017/039372
(87) International publication number: WO 2018/013332

(56) References cited:
- WO-A1-2012/075125
- WO-A1-2016/032589
- XIANG LI ET AL: "Dissecting meiotic recombination based on tetrad analysis by single-microspore sequencing in maize", NATURE COMMUNICATIONS, vol. 6, 24 March 2015 (2015-03-24), page 6648, XP055406201, DOI: 10.1038/ncomms7648 cited in the application
- Sateesh Kagale: "Single cell genomic sequencing in plants: current possibilities and limitations", , 1 December 2015 (2015-12-01), XP055406205, Retrieved from the Internet: URL:http://www.agwest.sk.ca/CIM-CID2015/SK agale_CID2015.pdf [retrieved on 2017-09-13]
- UJJAL KUMAR NATH ET AL: "Early, non-destructive selection of microspore-derived embryo genotypes in oilseed rape (Brassica napus L.) by molecular markers and oil quality analysis", MOLECULAR BREEDING, KLUWER ACADEMIC PUBLISHERS, DO, vol. 19, no. 3, 13 January 2007 (2007-01-13), pages 285-289, XP019482977, ISSN: 1572-9788, DOI: 10.1007/S11032-006-9053-Y
- FILIPPO M. BASSI ET AL: "Breeding schemes for the implementation of genomic selection in wheat ( Triticum spp . )", PLANT SCIENCE, vol. 242, 6 September 2015 (2015-09-06), pages 23-36, XP055406230, IE ISSN: 0168-9452, DOI: 10.1016/j.plantsci.2015.08.021

## Description

### FIELD OF INVENTION

The invention relates generally to the field of plant breeding and plant biotechnology.

### BACKGROUND

Pollen grains are produced in microsporangia contained within the anthers of a flower. During development, each anther forms two general groups of cells, the reproductive or sporogenous cells that give rise to microspores, and the non-reproductive cells. Two distinct and successive developmental phases lead to the production of mature pollen grains (or microgametophytes); these phases are known as microsporogenesis and microgametogenesis. During microsporogenesis, the diploid sporogenous cells differentiate into pollen mother cells (or microsporocytes) which then divide by meiosis to form four haploid tetrad microspores. The tetrad microspores are contained within a callose wall, which is later broken down by an enzyme called callase, freeing the tetrad microspores into the fluid-filled anther locule, which contains hundreds and thousands of individual microspores. Then, during microgametogenesis, the microspores undergo mitosis and develop into mature pollen grains, which contain the male gametes.

The meiotic crossing over that occurs during microsporogenesis results in the segregation of alleles into four recombinant haploid products (i.e. tetrad microspores, individual microspores, or later, gametes in the pollen grains). This re-assortment of alleles generates genetic diversity, which is desirable in plant breeding for the creation of new plant varieties. Knowing the genotype of the male gamete before fertilization would be advantageous because it would allow breeders to select pollen grains with desirable genetic compositions for fertilization with a female. However, there are no methods or means available to date to non-destructively genotype a microspore or a pollen grain such that it can be later used.

Moreover, plant breeding for a number of crops has been revolutionized by doubled haploid technology, in which haploid plant embryos (or plants produced therefrom) are obtained and then doubled, allowing the rapid production of recombinant lines with favorable gene combinations. Haploid embryos can be produced in vitro using either gynogenesis (embryo culture) or androgenesis (anther and microspore culture); however, androgenesis is the preferred method because it offers a much larger number of gametic cells with independent recombination events. Not all of the microspores have a favorable genetic constitution; thus, it would be beneficial to a plant breeder to know the genotype of a microspore so that decisions can be made early on in a breeding program on whether a microspore can be used to directly or indirectly produce another plant, part thereof, or cell culture (i.e. whether or not a microspore has a desirable genotype).

Therefore there is a need in the art for methods to non-destructively obtain the genotype of a microspore or pollen grain.

Ujjal Kumar Nath *et al.* (2007) discloses early, non-destructive selection of microspore-derived embryo genotypes in oilseed rape (*Brassica napus* L.) by molecular markers and oil quality analysis.

WO 2016/032589 A1 discloses systems and methods for genotyping plant material.

### SUMMARY

Methods for non-destructive genotyping a microspore are provided. The methods include obtaining genotypes in such a way such that the microspore can be later used (either directly or indirectly to develop a plant or plant part). The methods include using meiotically-related products to determine the genotype of another meiotically-related product in a non-destructive manner.

Methods for obtaining the genotype of a microspore in a non-destructive manner, such that the microspore can be later used, are provided herein. The methods include: isolating a microspore tetrad comprising four tetrad microspores, separating the microspore tetrad to obtain four tetrad microspores, genotyping three of the four tetrad microspores; and inferring the genotype of the fourth tetrad microspore from the genotypes of the other three based on allelic segregation. Staging may be performed to isolate the microspore tetrads. The microspore tetrad may be mechanically separated, such as with a micromanipulator, a cell sorter, or using microfluidics, or the microspore tetrad may be chemically separated, e.g. enzymatic digestion. The fourth tetrad microspore may be isolated after separation, and the three tetrad microspores to be genotyped may be lysed to obtain DNA using cold-heat shock, enzymatic digestion, DNA extraction, or any other method known to one of ordinary skill in the art. Whole genome amplification may also be performed prior to genotyping to increase the quantity and quality of the DNA from the three tetrad microspores. The three tetrad microspores may be genotyped individually, or qPCR may be applied to a pooled sample of the three tetrad microspores to examine copies of each allele. Once the genotype of the fourth tetrad microspore is inferred, the fourth tetrad microspore may be selected for further growth and development based on: a preferred genotype at least one locus, a whole genome genotype, a genome-wide genotype, at least one chromosome from a different species, a trait of interest including but not limited to simple and complex traits, a mutation, a gene knock-out, deletion, or silencing, a transgene locus, a recombinant haplotype, a genetic complement to another genotype, or any combination thereof. The selected fourth tetrad microspore may be placed in contact with a chromosome doubling agent such as but not limited to colchicine, in order to obtain a doubled microspore. A doubled haploid embryo may be produced from the doubled microspore, and the doubled haploid embryo may be grown into a doubled haploid plant. Alternatively, a haploid embryo may be produced from the selected fourth tetrad microspore, and the haploid embryo may be placed in contact with a chromosome doubling agent such as but not limited to colchicine to produce a doubled haploid embryo. The doubled haploid embryo may be grown into a doubled haploid plant. In still yet another aspect, a selected fourth tetrad microspore may be used to fertilize a female gametic cell.

Methods for making doubled haploid plants are also provided. One method involves isolating a microspore tetrad; separating the microspore tetrad to obtain four tetrad microspores; genotyping three of the four tetrad microspores; inferring the genotype of the fourth tetrad microspore based on the genotypes of the three tetrad microspores that are genotyped; culturing the fourth tetrad microspore; contacting the cultured fourth tetrad microspore with a chromosome doubling agent such as but not limited to colchicine; producing a doubled haploid embryo from the doubled haploid microspore; and generating a doubled haploid plant from the doubled haploid embryo. Another method involves: isolating a microspore tetrad; separating the microspore tetrad to obtain four tetrad microspores; genotyping three of the four tetrad microspores; inferring the genotype of the fourth tetrad microspore based on the genotypes of the three tetrad microspores that are genotyped ; producing a haploid embryo from the fourth tetrad microspore; contacting the haploid embryo with a chromosome doubling agent such as but not limited to colchicine; and growing the doubled haploid embryo into a doubled haploid plant. The genome of the haploid embryo or microspore may be edited in order to generate a favorable genetic composition or to introduce traits that facilitate further growth and development.

Methods for obtaining the genotype of a pollen grain in a non-destructive manner, such that it can be later used, are disclosed herein. The methods include obtaining four meiotically-related products from a pollen tetrad, genotyping three of the four meiotically-related products, inferring the genotype of the fourth meiotically-related product from the genotypes of the other three; and growing the fourth meiotically-related product into a mature pollen grain. The four meiotically-related products may be mechanically separated from one another, such as with a micromanipulator, a cell sorter, or with microfluidics, or chemically separated, e.g. enzymatic digestion. Alternatively, one of ordinary skill in the art may take advantage of a mutation in a quartet gene, such as but not limited to the quartet1 (QRT1) gene. DNA may be obtained from the meiotically-related products using any method known to one of ordinary skill in the art. The three meiotically-related products may be genotyped individually or qPCR may be applied to a pooled sample of the three meiotically-related products. Once the genotype of the fourth meiotically-related product is inferred, the fourth meiotically-related product may be selected for further growth and development based on: a preferred genotype at at least one locus, a whole genome genotype, a genome-wide genotype, at least one chromosome from a different species, a trait of interest including but not limited to simple and complex traits, a mutation, a gene knock-out, deletion, or silencing, a transgene locus, a recombinant haplotype, a genetic complement to another genotype, or any combination thereof. This may include performing pollination with the pollen grain.

In any of the preceding methods, the plant may be maize or canola.

Viable genotyped microspores or pollen grains produced by any of these methods, as well as seed, cells, plants, germplasm, progeny, and plant parts derived therefrom are further disclosed.

Accordingly, the invention provides a method for non-destructively obtaining the genotype of a microspore, said method comprising:
(a) isolating a microspore tetrad comprising four tetrad microspores,
(b) separating the microspore tetrad to obtain four tetrad microspores,
(c) genotyping three of the four tetrad microspores; and
(d) inferring the genotype of the fourth tetrad microspore from the genotypes obtained in step (c).

The invention further provides a method of obtaining a microscope, comprising:
(i) carrying out steps (a) to (d) as described above; and
(ii) selecting a fourth tetrad microspore based on: a preferred genotype at at least one locus, a whole genome genotype, a genome-wide genotype, at least one chromosome from a different species, a trait of interest including but not limited to simple and complex traits, a mutation, a gene knock-out, deletion, silencing, a transgene locus, a recombinant haplotype, a genetic complement to another genotype, or any combination thereof.

Yet further provided by the invention is a method of obtaining a doubled microspore, comprising:
(i) carrying out steps (a) to (d) as described above;
(ii) selecting a fourth tetrad microspore based on: a preferred genotype at at least one locus, a whole genome genotype, a genome-wide genotype, at least one chromosome from a different species, a trait of interest including but not limited to simple and complex traits, a mutation, a gene knock-out, deletion, silencing, a transgene locus, a recombinant haplotype, a genetic complement to another genotype, or any combination thereof; and
(iii) placing the selected fourth tetrad microspore in contact with a chromosome doubling agent; preferably wherein said chromosome doubling agent is colchicine.

The invention further provides a method of producing a doubled haploid embryo or doubled haploid plant, comprising:
(i) carrying out steps (a) to (d) as described above;
(ii) selecting a fourth tetrad microspore based on: a preferred genotype at at least one locus, a whole genome genotype, a genome-wide genotype, at least one chromosome from a different species, a trait of interest including but not limited to simple and complex traits, a mutation, a gene knock-out, deletion, silencing, a transgene locus, a recombinant haplotype, a genetic complement to another genotype, or any combination thereof;
(iii) placing the selected fourth tetrad microspore in contact with a chromosome doubling agent; preferably wherein said chromosome doubling agent is colchicine; and
(iv) producing a doubled haploid embryo from the doubled microspore;
optionally further comprising:
(v) growing the doubled haploid embryo into a doubled haploid plant.

Yet further provided by the invention is a method of producing a haploid embryo from a microspore, comprising:
(i) carrying out steps (a) to (d) as described above;
(ii) selecting a fourth tetrad microspore based on: a preferred genotype at at least one locus, a whole genome genotype, a genome-wide genotype, at least one chromosome from a different species, a trait of interest including but not limited to simple and complex traits, a mutation, a gene knock-out, deletion, silencing, a transgene locus, a recombinant haplotype, a genetic complement to another genotype, or any combination thereof; and
(iii) producing a haploid embryo from the selected fourth tetrad microspore.

The invention further provides a method of producing a doubled haploid embryo, comprising:
(i) carrying out steps (a) to (d) as described above;
(ii) selecting a fourth tetrad microspore based on: a preferred genotype at at least one locus, a whole genome genotype, a genome-wide genotype, at least one chromosome from a different species, a trait of interest including but not limited to simple and complex traits, a mutation, a gene knock-out, deletion, silencing, a transgene locus, a recombinant haplotype, a genetic complement to another genotype, or any combination thereof;
(iii) producing a haploid embryo from the selected fourth tetrad microspore; and
(iv) placing the haploid embryo in contact with a chromosome doubling agent; preferably wherein said chromosome doubling agent is colchicine.

Yet further provided by the invention is a method for making a doubled haploid plant, said method comprising:
(a) isolating a microspore tetrad;
(b) separating the microspore tetrad to obtain four tetrad microspores;
(c) genotyping three of the four tetrad microspores;
(d) inferring the genotype of the fourth tetrad microspore from the genotypes obtained in step (c); and
   (I)
      (e) culturing the fourth tetrad microspore;
      (f) contacting the cultured fourth tetrad microspore with a chromosome doubling agent;
      (g) producing a doubled haploid embryo from the doubled microspore; and
      (h) generating a doubled haploid plant from the doubled haploid embryo; or
   (II)
      (e) producing a haploid embryo from the fourth tetrad microspore;
      (f) contacting the haploid embryo with a chromosome doubling agent; and
      (g) growing the doubled haploid embryo into a doubled haploid plant.

### BRIEF DESCRIPTION OF THE DRAWINGS

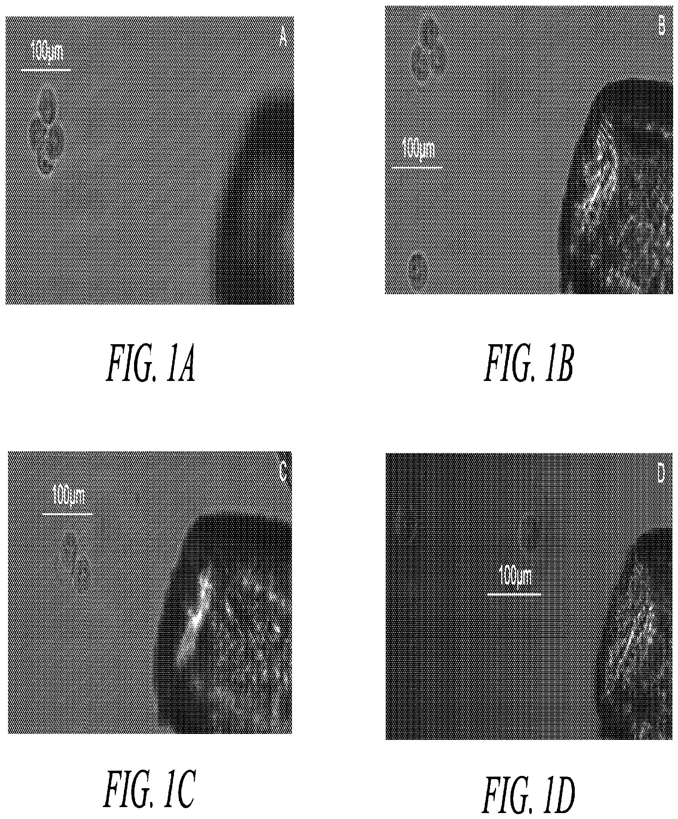

Figures 1A through 1D show the physical separation of the tetrad microspores from a maize tetrad. A micromanipulator was used to separate the tetrad microspores under a microscope. A single intact tetrad was isolated (Figure 1A), and the manipulator was used to separate the individual tetrad microspores from the tetrad structure one by one (Figures 1B through 1D)

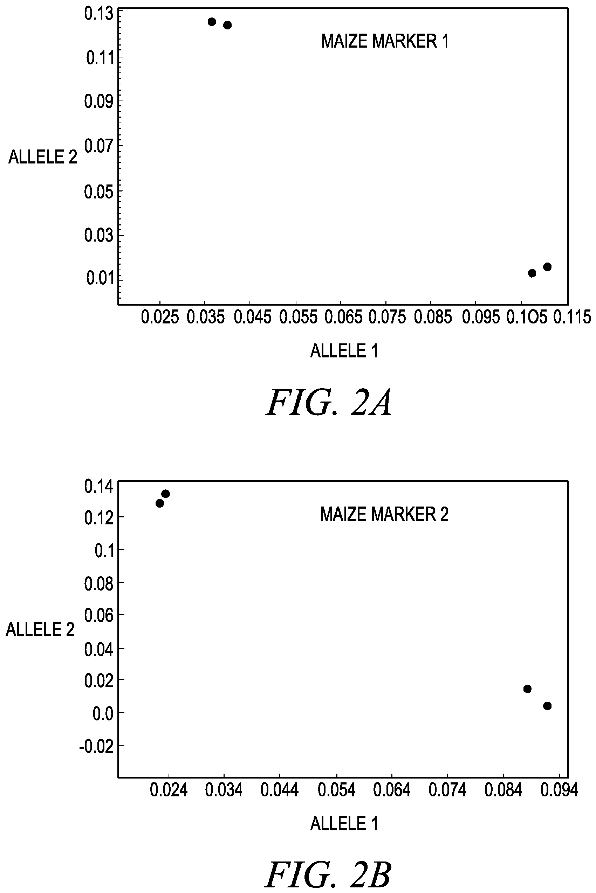

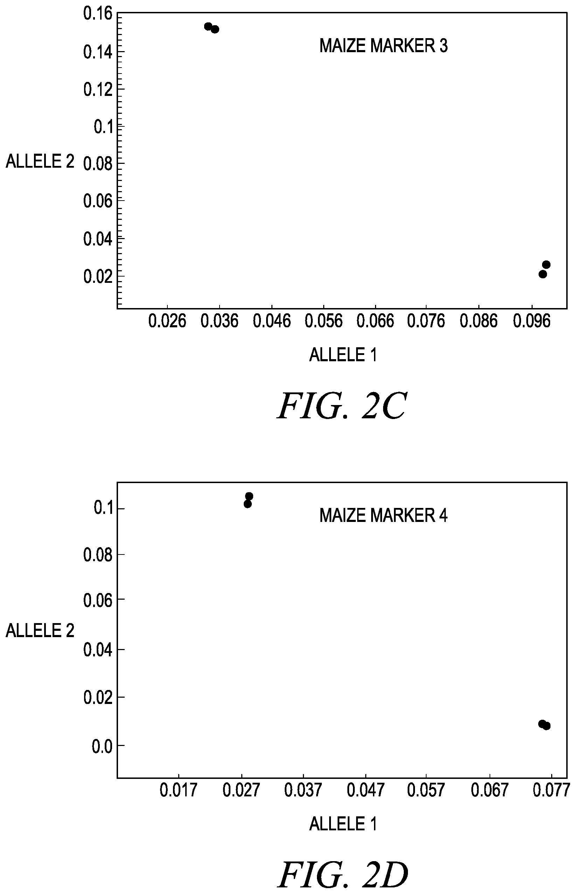

Figures 2A through 2D provide Taqman genotyping scatter plots for tetrad microspores separated from a tetrad. Each panel represents results from one marker. Figure 2A shows results for "Maize Marker 1"; Figure 2B shows results for "Maize Marker 2"; Figure 2C shows results for "Maize Marker 3"; and Figure 2D shows results for "Maize Marker 4". The results show an even distribution of paternal and maternal alleles (2:2 ratio) across the four tetrad microspores (when all four are destructively genotyped).

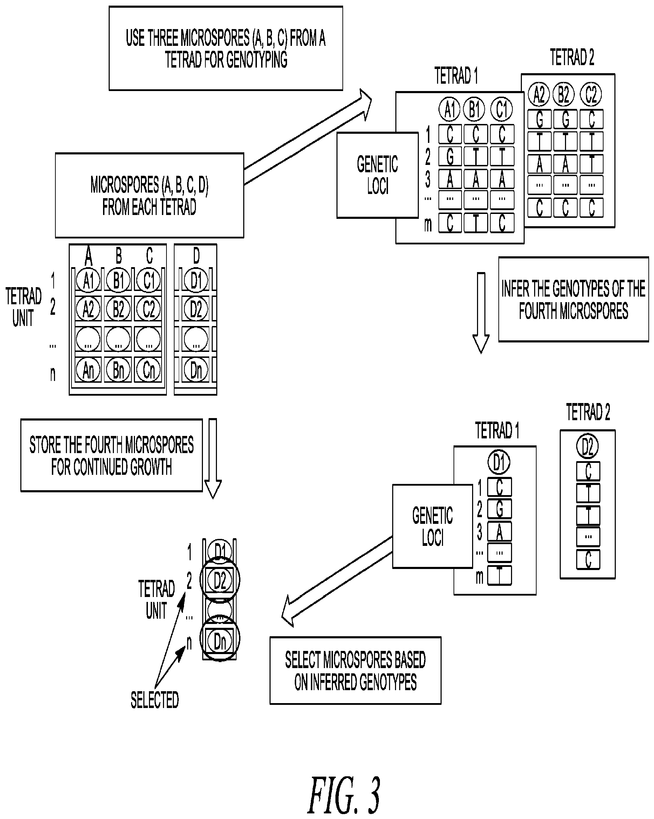

Figure 3 provides a schematic of the method to obtain the genotype of a fourth microspore from the genotypes of the other three microspores present in the same tetrad.

### DETAILED DESCRIPTION

As used in this specification and the appended claims, terms in the singular and the singular forms "a," "an," and "the," for example, include plural referents unless the content clearly dictates otherwise. Thus, for example, reference to "plant," "the plant," or "a plant" also includes a plurality of plants; also, depending on the context, use of the term "plant" can also include genetically similar or identical progeny of that plant; use of the term "a nucleic acid" optionally includes, as a practical matter, many copies of that nucleic acid molecule; similarly, the term "probe" optionally (and typically) encompasses many similar or identical probe molecules.

As used herein, the terms "comprises," "comprising," "includes," "including," "has," "having," "contains", "containing," "characterized by" or any other variation thereof, are intended to cover a non-exclusive inclusion, subject to any limitation explicitly indicated. For example, a composition, mixture, process, method, article, or apparatus that comprises a list of elements is not necessarily limited to only those elements but may include other elements not expressly listed or inherent to such composition, mixture, process, method, article, or apparatus.

The transitional phrase "consisting of" excludes any element, step, or ingredient not specified. In a claim, such would close the claim to the inclusion of materials other than those recited except for impurities ordinarily associated therewith. When the phrase "consisting of" appears in a clause of the body of a claim, rather than immediately following the preamble, it limits only the element set forth in that clause; other elements are not excluded from the claim as a whole. The transitional phrase "consisting essentially of" is used to define a composition, method or apparatus that includes materials, steps, features, components, or elements, in addition to those literally disclosed, provided that these additional materials, steps, features, components, or elements do not materially affect the basic and novel characteristic(s) of the claimed invention.
Certain definitions used in the specification and claims are provided below.

"Androgenesis" is the process by which haploid plants develop from the male gametophyte.

"Anther culture" is the process of culturing intact anthers.

"Allele" means any of one or more alternative forms of a genetic sequence. In a diploid cell or organism, the two alleles of a given sequence typically occupy corresponding loci on a pair of homologous chromosomes. With regard to a SNP marker, allele refers to the specific nucleotide base present at that SNP locus in that individual plant.

The term "amplifying" in the context of nucleic acid amplification is any process whereby additional copies of a selected nucleic acid (or a transcribed form thereof) are produced. An "amplicon" is an amplified nucleic acid, e.g., a nucleic acid that is produced by amplifying a template nucleic acid by any available amplification method.

"Callus" refers to a dedifferentiated proliferating mass of cells or tissue.

The phrases "contacting", "comes in contact with" or "placed in contact with" can be used to mean "direct contact" or "indirect contact". For example, the medium comprising a doubling agent may have direct contact with the haploid cell or the medium comprising the doubling agent may be separated from the haploid cell by filter paper, plant tissues, or other cells thus the doubling agent is transferred through the filter paper or cells to the haploid cell.

A "diploid" plant has two sets (genomes) of chromosomes and the chromosome number (2n) is equal to that in the zygote.

A doubled haploid or doubled haploid plant or cell, also referred to as a dihaploid or dihaploid plant or cell, is one that is developed by the doubling of a haploid set of chromosomes. A plant or seed that is obtained from a doubled haploid plant that is selfed any number of generations may still be identified as a doubled haploid plant. A doubled haploid plant is considered a homozygous plant. A plant is considered to be doubled haploid if it is fertile, even if the entire vegetative part of the plant does not consist of the cells with the doubled set of chromosomes. For example, a plant will be considered a doubled haploid plant if it contains viable gametes, even if it is chimeric.

A "doubled haploid embryo" is an embryo that has one or more cells that contain 2 sets of homozygous chromosomes.

A "gametic cell" is a reproductive cell having a haploid number of chromosomes.

A "genetic map" is a description of genetic association or linkage relationships among loci on one or more chromosomes (or linkage groups) within a given species, generally depicted in a diagrammatic or tabular form.

"Genotype" is a description of the allelic state at one or more loci in a genome.

"Gynogenesis" is the process by which haploid plants develop from the female gametophyte.

A "haploid" is a plant with the gametic or n number of chromosomes.

"Haplotype" refers to a combination of particular alleles present within a particular plant's genome at two or more linked marker loci, for instance at two or more loci on a particular linkage group. For instance, in one example, two specific marker loci on ch 1 are used to define a haplotype for a particular plant. In still further examples, 3, 4, 5, 6, 7, 8, 9, 10, 11, 12, 13, 14, 15, 16, 17, 18, 19, 20, or more linked marker loci are used to define a haplotype for a particular plant.

The terms "label" and "detectable label" refer to a molecule capable of detection. A detectable label can also include a combination of a reporter and a quencher, such as are employed in FRET probes or TAQMAN® probes. The term "reporter" refers to a substance or a portion thereof that is capable of exhibiting a detectable signal, which signal can be suppressed by a quencher. The detectable signal of the reporter is, e.g., fluorescence in the detectable range. The term "quencher" refers to a substance or portion thereof that is capable of suppressing, reducing, inhibiting, etc., the detectable signal produced by the reporter. As used herein, the terms "quenching" and "fluorescence energy transfer" refer to the process whereby, when a reporter and a quencher are in close proximity, and the reporter is excited by an energy source, a substantial portion of the energy of the excited state nonradiatively transfers to the quencher where it either dissipates nonradiatively or is emitted at a different emission wavelength than that of the reporter.

A "male gametic cell" as used herein is any male haploid cell involved in the process of microsporogenesis and microgametogenesis. A male gametic cell may comprise but is not limited to a tetrad microspore, a single cell microspore, or a pollen grain. The term "male gametic cell" may also comprise tetrad pollen grains found in the quartet mutants.

"Marker" or "molecular marker" is a term used to denote a nucleic acid or amino acid sequence that is sufficiently unique to characterize a specific locus on the genome. Any detectible polymorphic trait can be used as a marker so long as it is inherited differentially and exhibits linkage disequilibrium with a phenotypic trait of interest.

As used herein, a "marker profile" means a combination of particular alleles present within a particular plant's genome at two or more marker loci which are not linked, for instance two or more loci on two or more different linkage groups or two or more chromosomes. For instance, in one example, one marker locus on chromosome 1 and a marker locus on another chromosome are used to define a marker profile for a particular plant. In certain other examples a plant's marker profile comprises one or more haplotypes.

The term "medium" includes compounds in liquid, gas, or solid state.

A "meiotically-related product" is a product of meiosis that occurs as a result of microsporogenesis. The meiotically-related product may be a microspore.

A "microspore" is an individual haploid structure produced from diploid sporogenous cells (microsporoyte, pollen mother cell, or meiocyte) following meiosis.

"Microspore culture", sometimes referred to as "pollen culture", is the process of isolating microspores from anthers before culture.

A "Microspore tetrad" as used herein is a single structure comprised of four individual physically attached tetrad microspores.

"Non-destructive genotyping" of a male gametic cell, as used herein, refers to a process for determining the genotype of a male gametic cell in a manner that preserves the viability of the male gametic cell for further development. For example, for non-destructive genotyping of a microspore, the microspore may be used for development of a plant embryo or for fertilization of a female gametic cell, or may just remain viable.

A "pollen grain" is a mature gametophyte containing vegetative (non-reproductive) cells and a generative (reproductive) cell.

As used herein, the term "plant" includes reference to whole plants, plant organs (e.g., leaves, stems, roots, etc.), seeds and plant cells and progeny of same. "Plant cell", as used herein includes, without limitation, seeds, suspension cultures, embryos, meristematic regions, callus tissue, leaves, roots, shoots, gametophytes, sporophytes, pollen, and microspores.

A "pollen tetrad" as used herein is a single structure comprised of four individual physically attached pollen grains.

"Polymorphism" means a change or difference between two related nucleic acids. A "nucleotide polymorphism" refers to a nucleotide that is different in one sequence when compared to a related sequence when the two nucleic acids are aligned for maximal correspondence.

"Polynucleotide," "polynucleotide sequence," "nucleic acid sequence," "nucleic acid fragment," and "oligonucleotide" are used interchangeably herein to indicate a polymer of nucleotides that is single- or multi-stranded, that optionally contains synthetic, non-natural, or altered RNA or DNA nucleotide bases. A DNA polynucleotide may be comprised of one or more strands of cDNA, genomic DNA, synthetic DNA, or mixtures thereof.

"Primer" refers to an oligonucleotide which is capable of acting as a point of initiation of nucleic acid synthesis or replication along a complementary strand when placed under conditions in which synthesis of a complementary strand is catalyzed by a polymerase. Typically, primers are about 10 to 30 nucleotides in length, but longer or shorter sequences can be employed. Primers may be provided in doublestranded form, though the single-stranded form is more typically used. A primer can further contain a detectable label, for example a 5' end label.

"Probe" refers to an oligonucleotide that is complementary (though not necessarily fully complementary) to a polynucleotide of interest and forms a duplexed structure by hybridization with at least one strand of the polynucleotide of interest. Typically, probes are oligonucleotides from 10 to 50 nucleotides in length, but longer or shorter sequences can be employed. A probe can further contain a detectable label.

A "tetrad" is used herein to refer to a single structure comprised of four individual tetrad microspores.or four individual physically attached pollen grains.

A "tetrad microspore" as used herein is one of four microspore members belonging to a tetrad. The tetrad microspores may or may not be physically associated (i.e. enclosed within the callose wall).

Standard recombinant DNA and molecular cloning techniques used herein are well known in the art and are described more fully in Sambrook et al. Molecular Cloning: A Laboratory Manual; Cold Spring Harbor Laboratory Press: Cold Spring Harbor, 1989 (hereinafter "Sambrook").

Methods are provided herein for obtaining the genotype of a male gamete at an early stage, for example the tetrad microspore stage, by inferring its genotype based on the genotypes of its related meiotic products from microsporogenesis. Once the genotype of a meiotically-related product is obtained, decisions can be made about its further use as part of a breeding program.

### Isolation and dissection of tetrads

Tetrads can be isolated by dissecting anthers at the appropriate stage of pollen development. Tetrads may be isolated from anthers using a variety of manual methods known in the art. For example, pollen-bearing flowers can be immersed in liquid, and tetrads can be isolated manually by pipetting dilutions until individual tetrads are obtained (see, e.g., Berchowitz & Copenhaver (2008) Nat Protoc 3:41-50; Li, X. and Yan J. 2015. Nature Communications 10.1038/ncomms7648). It should be noted that tetrads from many species will not remain intact if only water is used, and an appropriate buffer may be required, such as, for example, 27% D-sorbitol solution.

Staging may be necessary to identify the correct developmental stage based on the tassel position. An anther locule may contain tetrads, naturally released single cell microspores, or both depending on developmental stage. Anthers containing tetrads are in the earlier developmental stage compared to anthers containing naturally released single cell microspores. In maize, for example, anthers collected from tassels can be placed in media and crushed to release the microspores. The released cells can be observed under a microscope to determine presence or absence of microspore tetrads. The sampling and observation steps may be repeated as part of the staging process to search for anthers containing tetrads.

Plants that naturally produce pollen in the form of a tetrad structure can also be used to easily isolate tetrads after dehiscence (see, e.g., Copenhaver (2005) J NC Acad Sci 121:17-35). Alternatively, genes such as but not limited to QRT1 can be mutated or silenced to produce meiotically-related pollen tetrads that are not physically connected. Mutation of QRT1 can be produced in inbred plants, or in hybrid plants (e.g., F1 generation) using technologies including but not limited to mutagenesis (e.g., EMS), transposons, RNAi-mediated silencing, transcriptional silencing, CRISPR/CAS, Zn Fingers, meganucleases TALENs, or any combination thereof.

Once a sample containing appropriate stage tetrads is obtained, the cells in the sample may be sorted to remove any single cell microspores (later stage). Individual tetrads may then be placed in separate compartments (such as, for example, in a plate) to reduce contamination. One or more of the tetrad isolation steps may be automated; including but not limited to the use of a cell sorter, a flow cytometer, microfluidics, a centrifuge, laser-mediated manipulation such as laser pressure catapulting, or any other matching and/or technique known in the art. For example, microfluidics may be used to position individual tetrads into singular compartments.

The next step is the separation or isolation of the cells in the tetrad structure to obtain four isolated tetrad microspores. Genes such as the QUARTET (QRT) genes, for example, are required for separation in some species. For example, in *Arabidopsis thaliana,* the QRT1, QRT2, and QRT3 genes are required for normal pollen development, and loss of function of any of them disrupts separation of pollen grains following meiosis (see, e.g., Francis et al. (2006) Plant Physiol 142:1004-1013). Thus, mutations in QRT genes that affect the integrity of the membrane enclosing the four meiotically-related products may be used to facilitate isolation of the individual cells. Mutations in QRT1 genes could be induced or directed in an inbred or in a hybrid (e.g., F1 generation) plant using technologies such as but not limited to mutagenic treatments (e.g., EMS), transposons, RNAi-mediated silencing, transcriptional silencing, CRISPR/CAS, Zn Fingers, meganucleases, TALENs, and any combination thereof.

In most cases, the meiotically-related products in a tetrad do remain physically attached for a significant period of time in wild-type plants and thus can be isolated and used in the methods provided herein. A tetrad may be dissected using chemical or mechanical methods. Chemical methods may include enzymatic degradation (e.g., of callose) or incubation with a detergent or acid, essentially any chemical that will break the cell wall without damaging the interior components. Mechanical methods may include agitation, mechanical force, repeated aspirations, or any combination thereof. In some examples, a micromanipulator may be used. Again, one or more of the steps to separate the individual meiotically-related products may be automated, including but not limited to the use of a cell sorter, a flow cytometer, microfluidics, a centrifuge, laser-mediated manipulation such as laser pressure catapulting, or any other machine and/or technique known in the art.

The tetrad may be from any plant having the tetrad structure (i.e. an angiosperm), such as but not limited to maize, rice, soybean, wheat, sorghum, millet, sugarcane, rye, barley, oat, canola, sunflower, cotton, soybean, or alfalfa.

### Genotyping

Three of the four meiotically-related products (e.g. tetrad microspores) may be genotyped using any method known to one of skill in the art, while the fourth may be isolated for future use, such as but not limited to storage, for microspore derived embryogenesis, or for pollination.

In order for the three meiotically-related products (e.g. microspores) to be genotyped, genetic material must be freed such that it is accessible for molecular analysis. This may involve physical treatments such as exposure to cold-heat or just heat, incubation with enzymes, or even DNA extraction techniques (although it is important to note that extraction is not a necessary step in obtaining DNA for molecular analysis). Essentially any process that disrupts the tissue and breaks open cells, thereby releasing DNA that can be used for molecular characterization, may be used in the methods provided herein. In some examples, whole genome amplification may be used; the Qiagen REPLI-g® kit, the Sigma-Aldrich SeqPlex kit, or any other technique known to one of ordinary skill in the art may be used for whole genome amplification.

Genotyping may be done using any method known to one of ordinary skill in the art, including but not limited to sequence-based methods (genome-wide or targeted), exome capture, hybridization methods (liquid, array, and solid support), as well as amplification based methods, including but not limited to polymerase chain reaction (PCR, e.g., Taqman), molecular probes (e.g., molecular inversion probes).

Any relevant genetic marker or set of genetic markers can be used to genotype the meiotically-related product(s). This may include whole genome sequencing, southern by sequencing, a genome-wide marker set, a chromosome marker set, a trait marker set, imputation, or any combination thereof. Further, any suitable type of marker can be used, including Restriction Fragment Length Polymorphisms (RFLPs), Single Sequence Repeats (SSRs), Target Region Amplification Polymorphisms (TRAPs), Isozyme Electrophoresis, Randomly Amplified Polymorphic DNAs (RAPDs), Arbitrarily Primed Polymerase Chain Reaction (AP-PCR), DNA Amplification Fingerprinting (DAF), Sequence Characterized Amplified Regions (SCARs), Amplified Fragment Length Polymorphisms (AFLPs), and Single Nucleotide Polymorphisms (SNPs).

In some examples, the three meiotically-related products (e.g. tetrad microspores) are genotyped as a pool or as sub-pool(s) to determine the ratio of the possible alleles, in other examples, the three meiotically-related products are each genotyped individually. For example, when the one meiotically-related product is removed from the tetrad structure and isolated, the remaining three may remain physically attached or associated, or may be separated into three individual meiotically-related products, and genotyped without further separation.

The genotype of the fourth meiotically related product (e.g. the fourth tetrad microspore) can then be inferred from the genotypes of the other three meiotically-related products based on allelic segregation. In sexually reproducing organisms, meiosis produces haploid gametes from parental diploid cells during the process of sexual reproduction. When a diploid cell undergoes meiosis to produce four haploid cells, exactly half of the genome in the each of the four cells should be maternal and the other half should be paternal. Consequently, for the four meiotically-related products (e.g. four tetrad microspores), a 2:2 ratio of paternal to maternal alleles is typically observed. There are rare cases in which the standard rules of genetics have been violated, however, due to gene conversion.

Deduction of the genotype of the fourth meiotically-related product in the tetrad typically involves the use and/or determination of polymorphisms such as sequence-based polymorphisms such as single nucleotide polymorphisms (SNPs), small insertion/deletions (Indels), presence/absence of transgenes, copy number variations, as well as non-sequence based marks (epigenetic marks) such as DNA methylation, and acetylation, that may be used individually or in any combination.

In a doubled haploid breeding program involving embryos generated via androgenesis, microspores may be selected for embryo culture based on their genotype. Additionally, pollen grains may be selected for manual pollination. Thus, in some examples, the methods comprise identifying a microspore for embryo culture or identifying a pollen grain for pollination by assaying for: a preferred genotype at at least one locus, a whole genome genotype, a genome-wide genotype, at least one chromosome from a different species, a trait of interest including but not limited to simple and complex traits, a mutation, a gene knock-out, deletion, or silencing, a transgene locus, a recombinant haplotype, a genetic complement to another genotype, or any combination thereof. In some examples, the preferred genotype is a genotype wherein a genetic region is absent or silenced. In some examples, the genetic region is silenced and the preferred genotype relates to whether the region's methylation is altered, increased or decreased.

### Selection, microspore embryogenesis, and generation of a plant

If the fourth meiotically-related product has a favorable genetic composition, it may be selected for further growth and development. Optionally, it may be targeted for gene editing using CRISPR/CAS, Zn Fingers, meganucleases, TALENs, or any combination thereof, to either generate a favorable genetic composition in a specific region of the genome or to introduce characteristics or traits that facilitate further growth and development.

In the case of microspores, any method known to one of ordinary skill in the art may be used to generate a maize plant from the cultured microspores, such as but not limited to Genovesi and Collins. 1982. Crop Sci. 22:1137; Jager, K. et al. 2015. Plant Cell Tiss Organ Cult 123:257-271; US Patent 5,322,789; and US Patent 5,602,310.

In further methods, the chromosomes may be doubled at the microspore stage, at the embryo stage, at the mature seed stage, or anytime between pollination of the plant and before the germination of the haploid seed. Alternatively, spontaneous doubling may also occur.

At the microspore stage, the microspores may be treated with a diploidization agent in order to obtain a doubled haploid maize embryo, which can then be grown into a doubled haploid maize plant. For instance, microspores may be placed in contact with a chromosome doubling agent such as colchicine or herbicides like amiprophos methyl, oryzalin, and pronamide (Hantzschel and Weber (2010) Protoplasma 241:99-104). A chromosome doubling agent may also be applied to multicellular clusters, pro-embryoids, or somatic embryos (any actively dividing cell).

A microspore selected using the methods provided herein may also be used to fertilize a female gametic cell.

In the case of pollen grains, if selected, a pollen grain can be used for pollination, enabling the fertilization of a female gamete and the development of a seed that can be grown into a plant.

### EXAMPLES

The present invention is illustrated by the following examples.

### Example 1 -Microspore Tetrad Isolation and Dissection

Maize tassels were collected from the greenhouse and stored at 10°C prior to isolation. Using a sterilized set of tweezers, a spikelet from one of the tassels was split open and the anthers extracted. The anthers were then placed in one well of a 96 well culture plate containing pre-culture media. Once in the media, the anthers were crushed to release the microspores. The crushed anthers were viewed under a microscope in order to identify tetrads. If only single cell microspores were present, anthers were then collected from a region of the tassel closer to the base. If no intact cells were visible, anthers were collected away from the base of the tassel. The sampling and observation steps were repeated until tetrads were identified.

A micromanipulator connected to a microscope was used to move and separate the tetrads. The micromanipulator tip is a plastic tip that was bent to accommodate the 96 well culture plate format. Individual tetrads were collected with the micromanipulator and transferred to a fresh well of a 96 well culture plate containing pre-culture media. The tetrad was then picked up again using the micromanipulator and transferred to another fresh well containing pre-culture media. The washing step was repeated once more, resulting in a total of three rinses prior to tetrad separation.

Once isolated and washed, the cells of the microspore tetrad were mechanically separated using the micromanipulator (Figures 1A through 1D). The following were used to accomplish separation:
- Aspiration: pulling and pushing the tetrad in and out of the micromanipulator tip until cells were separated from the tetrad.
- "Rolling": using the micromanipulator to push the tetrad along the bottom of the culture plate to dislodge loose cells from the tetrad.
- "Press and drag": bringing the tip of the micromanipulator down directly onto the tetrad, applying pressure to push it against the base of the plate, and then moving the micromanipulator horizontally to drag the tetrad along the bottom of the plate.

In another method, enzymatic digestion with β -1,3-glucanase was used to digest the tetrad callose walls. Tetrads were transferred into individual wells of a cell culture plate containing 100µL 0.6M mannitol pre-culture media using a Nikon Eclipse T-IE. Two treatment conditions were tested. In the first treatment, 100µL of 0.6M mannitol pre-culture media containing β -1,3-glucanase (Sigma-Aldrich #67138) was added to each well for a final concentration of 1.5 mg/mL glucanase (∼0.3U/mL). In the second treatment, 100µL of 0.6M mannitol pre-culture media without enzyme was added to each well. Observations were made 1) at the beginning of the experiment before the treatment; 2) after being covered in aluminum foil and incubated at room temperature (21°C) overnight on a rocker plate set to high; and 3) after the 2nd observation, when all tetrads were agitated by gently pipetting the media in and out with a 200uL pipettor. The addition of β-1,3-glucanase to pre-culture media sufficiently degraded the callose wall of a tetrad, separating the microspores, following an overnight incubation and gentle agitation with a pipettor. Without agitation, all tetrads remained intact. Tetrads remained intact when β-1,3-glucanase was not added.

Once separated, a 2.5 uL pipette was used to transfer individual microspores from the culture plate to PCR strip tubes containing sterile TE buffer. Tubes were kept on ice during handling, and the contents were used immediately in Whole Genome Amplification, or stored at -80°C.

### Example 2 - Microspore Genotyping

DNA was amplified from three of the four microspore cells using whole genome amplification (Qiagen's REPLI-g Single Cell WGA kit). All whole genome amplification steps were performed in a laminar flow hood. Following whole genome amplification, the samples were diluted 1:100 in TE buffer and the samples were genotyped using Quanta ToughMix Custom Mix #7 and 8 internal markers. Genotyping was performed using a modified Array Tape program on a Quantstudio 7 Flex real-time thermocycler. Genotyping calls were made using Quantstudio 6 and 7 Flex Software.

Once the genotypes of the microspores were obtained, the genotype of the remaining microspore was inferred from the genotypes of the other microspores based on chromosome recombination and allelic segregation. The resulting whole genome amplified product was used as a DNA template for surveying specific loci of interest within the genome (Figures 2A through 2D), or for performing whole genome-wide analysis using targeted genotype-by-sequencing.

For example, in Figure 3, for tetrad 1, microspore A1 has a "C" at genetic locus 1; microspore B1 has a "C" at genetic locus 1; and microspore C1 has a "C" at genetic locus 1. Thus, it can be inferred that the fourth microspore, D1, also has a "C" at genetic locus 1. At genetic locus 2, microspore A1 has a "G"; microspore B1 has a "T"; and microspore C1 has a "T". Thus, it can be inferred that microspore D1 has a "G".

### Example 3 - Selection and Path to Plant

If the fourth microspore is selected for further growth and development (such as to generate a plant or plant part) based on the genotypic analysis, the microspore can be subjected to a chromosome doubling treatment, such as colchicine. The doubled microspore can then be grown into a doubled haploid embryo and then a doubled haploid plant. Alternatively, the microspore can be cultured using standard embryo rescue techniques and then placed in contact with a chromosome doubling agent at the embryo stage, at the mature seed stage, or anytime between pollination of the plant and before the germination of the haploid seed. Alternatively, spontaneous doubling may also occur.

In addition, the genome of the microspore may be edited prior to embryogenesis, as a haploid, using CRISPR/CAS, Zn Fingers, meganucleases, TALENs, or any combination thereof, to generate a favorable genetic composition in a specific region of the genome or to introduce genes that confer traits that facilitate further growth and development.

### Example 4 - Obtaining the Genotype of a Pollen Grain

Pollen tetrads can be isolated by dissecting anthers at the appropriate stage of pollen development using any of a variety of manual methods known in the art. For example, pollen-bearing flowers can be immersed in liquid to allow the pollen tetrads to be suspended into the solution. Pollen tetrads can then be isolated manually by pipetting dilutions (see, e.g., Berchowitz & Copenhaver (2008) Nat Protoc 3:41-50). It should be noted that pollen from many species will not remain intact if only water is used, and an appropriate buffer may be required. One or more of the isolation steps can be automated. For example, pollen tetrads can be isolated using microfluidics. Alternatively, plants with pollen quartets can be used to easily isolate quartets after dehiscence.

Once pollen tetrads have been isolated, the four meiotically-related pollen grains can be separated from one another using chemical or mechanical means and three of the four isolated male gametophytes can be genotyped using any known method, including but not limited to sequence-based methods (genome-wide or targeted), exome capture, hybridization methods (liquid, array, and solid support), as well as amplification based methods, including but not limited to polymerase chain reaction (PCR, e.g., Taqman), molecular probes (e.g., molecular inversion probes). The genotype of the fourth male gametophyte can be inferred based on the genotypes of its meiotically-related products.

### Example 5 -Microspore Tetrad Isolation and Dissection in Canola

Microspore tetrads from dicots, such as canola, can be isolated using any method known to the one of ordinary skill in the art. Microspore tetrads from a canola plant, for example, can be separated either mechanically or chemically to obtain four microspores from a tetrad, and three of the four tetrad microspores can be genotyped as described in Example 2. The genotype of the fourth tetrad microspore can be non-destructively obtained by inferring the genotype based on the expected 2:2 ratio. A canola embryo can then be obtained from the fourth microspore if selected for further growth and development, and the embryo can be grown into a canola plant.

## Claims

1. A method for non-destructively obtaining the genotype of a microspore, said method comprising:
(a) isolating a microspore tetrad comprising four tetrad microspores,
(b) separating the microspore tetrad to obtain four tetrad microspores,
(c) genotyping three of the four tetrad microspores; and
(d) inferring the genotype of the fourth tetrad microspore from the genotypes obtained in step (c).

2. The method of claim 1, wherein the microspore tetrad is from:
(i) maize; or
(ii) canola.

3. The method of claim 1, wherein the microspore tetrad is mechanically separated in step (b) to obtain four tetrad microspores; preferably wherein:
(i) a micromanipulator or cell sorter; or
(ii) a microfluidics system
is used to mechanically separate the microspore tetrad.

4. The method of claim 1, wherein the microspore tetrad is enzymatically separated in step (b) to obtain four tetrad microspores.

5. The method of claim 1, further comprising isolating the fourth tetrad microspore after step (b).

6. The method of claim 1:
(i) wherein prior to genotyping the three tetrad microspores, whole genome amplification is performed; or
(ii) wherein the three tetrad microspores are genotyped individually or qPCR is applied to a pooled sample of the three tetrad microspores.

7. A method of obtaining a microspore, comprising:
(i) carrying out steps (a) to (d) of claim 1; and
(ii) selecting the fourth tetrad microspore based on: a preferred genotype at at least one locus, a whole genome genotype, a genome-wide genotype, at least one chromosome from a different species, a trait of interest including but not limited to simple and complex traits, a mutation, a gene knock-out, deletion, silencing, a transgene locus, a recombinant haplotype, a genetic complement to another genotype, or any combination thereof.

8. A method of obtaining a doubled microspore, comprising:
(i) carrying out steps (i) and (ii) of claim 7; and
(ii) placing the selected fourth tetrad microspore in contact with a chromosome doubling agent; preferably wherein said chromosome doubling agent is colchicine.

9. A method of producing a doubled haploid embryo or doubled haploid plant, comprising:
(i) carrying out steps (i) and (ii) of claim 8; and
(ii) producing a doubled haploid embryo from the doubled microspore; optionally further comprising:
(iii) growing the doubled haploid embryo into a doubled haploid plant.

10. A method of producing a haploid embryo from a microspore, comprising:
(i) carrying out steps (i) and (ii) of claim 7; and
(ii) producing a haploid embryo from the selected fourth tetrad microspore.

11. A method of producing a doubled haploid embryo, comprising:
(i) carrying out steps (i) and (ii) of claim 10; and
(ii) placing the haploid embryo in contact with a chromosome doubling agent; preferably wherein said chromosome doubling agent is colchicine.

12. A method for making a doubled haploid plant, said method comprising:
(a) isolating a microspore tetrad;
(b) separating the microspore tetrad to obtain four tetrad microspores;
(c) genotyping three of the four tetrad microspores;
(d) inferring the genotype of the fourth tetrad microspore from the genotypes obtained in step (c); and
(I)
(e) culturing the fourth tetrad microspore;
(f) contacting the cultured fourth tetrad microspore with a chromosome doubling agent;
(g) producing a doubled haploid embryo from the doubled microspore; and
(h) generating a doubled haploid plant from the doubled haploid embryo; or
(II)
(e) producing a haploid embryo from the fourth tetrad microspore;
(f) contacting the haploid embryo with a chromosome doubling agent; and
(g) growing the doubled haploid embryo into a doubled haploid plant.

13. The method of claim 12, wherein:
(a) said microspore tetrad is from:
(i) maize; or
(ii) canola;
(b) said chromosome doubling agent is colchicine; or
(c) the genome of the fourth tetrad microspore is edited prior to step (I)(f) to generate a favorable genetic composition or to introduce traits that facilitate further growth and development.

14. The method of claim 12(II), wherein the genome of the haploid embryo is edited prior to step (II)(f) to generate a favorable genetic composition or to introduce traits that facilitate further growth and development.

## Patentansprüche

1. Verfahren zum nicht-destruktiven Erhalten des Genotyps einer Mikrospore, wobei das Verfahren umfasst:
(a) Isolieren einer Mikrosporen-Tetrade, die vier Tetraden-Mikrosporen umfasst,
(b) Trennen der Mikrosporen-Tetrade, um vier Tetraden-Mikrosporen zu erhalten,
(c) Genotypisierung von drei der vier Tetraden-Mikrosporen; und
(d) Ableiten des Genotyps der vierten Tetraden-Mikrospore aus den in Schritt (c) erhaltenen Genotypen.

2. Verfahren nach Anspruch 1, wobei die Mikrosporen-Tetrade stammt von:
(i) Mais; oder
(ii) Raps.

3. Verfahren nach Anspruch 1, wobei die Mikrosporen-Tetrade in Schritt (b) mechanisch getrennt wird, um vier Tetraden-Mikrosporen zu erhalten; vorzugsweise wobei:
(i) ein Mikromanipulator oder Zellsortierer; oder
(ii) ein Mikrofluidiksystem
verwendet wird, um die Mikrosporen-Tetrade mechanisch zu trennen.

4. Verfahren nach Anspruch 1, wobei die Mikrosporen-Tetrade in Schritt (b) enzymatisch getrennt wird, um vier Tetraden-Mikrosporen zu erhalten.

5. Verfahren nach Anspruch 1, das ferner das Isolieren der vierten Tetrade-Mikrospore nach Schritt (b) umfasst.

6. Verfahren nach Anspruch 1:
(i) wobei vor der Genotypisierung der drei Tetraden-Mikrosporen eine Ganzgenom-Amplifikation durchgeführt wird; oder
(ii) wobei die drei Tetraden-Mikrosporen einzeln genotypisiert werden oder qPCR auf eine gepoolte Probe der drei Tetraden-Mikrosporen angewendet wird.

7. Verfahren zum Erhalten einer Mikrospore, umfassend:
(i) Ausführen der Schritte (a) bis (d) nach Anspruch 1; und
(ii) Auswählen der vierten Tetraden-Mikrospore basierend auf: einem bevorzugten Genotyp an wenigstens einem Locus, einem Gesamtgenom-Genotyp, einem genomweiten Genotyp, wenigstens einem Chromosom aus einer anderen Spezies, einem Merkmal von Interesse, einschließlich, jedoch ohne darauf beschränkt zu sein, einfacher und komplexer Merkmale, einer Mutation, eines Gen-Knock-outs, Deletion, Silencing, eines Transgen-Locus, eines rekombinanten Haplotyps, eines genetischen Komplement zu einem anderen Genotyp oder jeder Kombination davon.

8. Verfahren zum Erhalten einer doppelten Mikrospore, umfassend:
(i) Ausführen der Schritte (i) und (ii) nach Anspruch 7; und
(ii) Inberührungbringen der ausgewählten vierten Tetraden-Mikrospore mit einem Chromosomenverdopplungsmittel; vorzugsweise wobei das Chromosomenverdopplungsmittel Colchicin ist.

9. Verfahren zum Herstellen eines doppelten haploiden Embryos oder einer doppelten haploiden Pflanze, umfassend:
(i) Ausführen der Schritte (i) und (ii) nach Anspruch 8; und
(ii) Herstellen eines doppelten haploiden Embryos aus der doppelten Mikrospore;
optional ferner umfassend:
(iii) Züchten des doppelten haploiden Embryos zu einer doppelten haploiden Pflanze.

10. Verfahren zum Herstellen eines haploiden Embryos aus einer Mikrospore, umfassend:
(i) Ausführen der Schritte (i) und (ii) nach Anspruch 7; und
(ii) Herstellen eines haploiden Embryos aus der ausgewählten vierten Tetraden-Mikrospore.

11. Verfahren zum Herstellen eines doppelt haploiden Embryos, umfassend:
(i) Ausführen der Schritte (i) und (ii) nach Anspruch 10; und
(ii) Inberührungbringen des haploiden Embryos mit einem Chromosomenverdopplungsmittel; vorzugsweise wobei das Chromosomenverdopplungsmittel Colchicin ist.

12. Verfahren zum Herstellen einer doppelten haploiden Pflanze, wobei das Verfahren umfasst:
(a) Isolieren einer Mikrosporen-Tetrade;
(b) Trennen der Mikrosporen-Tetrade, um vier Tetraden-Mikrosporen zu erhalten;
(c) Genotypisierung von drei der vier Tetraden-Mikrosporen;
(d) Ableiten des Genotyps der vierten Tetraden-Mikrospore aus den in Schritt (c) erhaltenen Genotypen; und
(I)
(e) Kultivieren der vierten Tetraden-Mikrospore
(f) Inberührungbringen der kultivierten vierten Tetraden-Mikrospore mit einem Chromosomenverdopplungsmittel;
(g) Herstellen eines doppelten haploiden Embryos aus der doppelten Mikrospore; und
(h) Erzeugen einer doppelten haploiden Pflanze aus dem doppelten haploiden Embryo; oder
(II)
(e) Herstellen eines haploiden Embryos aus der vierten Tetraden-Mikrospore;
(f) Inberührungbringen des haploiden Embryos mit einem Chromosomenverdopplungsmittel; und
(g) Züchten des doppelten haploiden Embryos zu einer doppelten haploiden Pflanze.

13. Verfahren nach Anspruch 12, wobei:
(a) die Mikrosporen-Tetrade stammt von:
(i) Mais; oder
(ii) Raps;
(b) das Chromosomenverdopplungsmittel Colchicin ist; oder
(c) das Genom der vierten Tetraden-Mikrospore vor Schritt (I)(f) editiert wird, um eine vorteilhafte genetische Zusammensetzung zu erzeugen oder Eigenschaften einzuführen, die das weiteres Wachstum und die weitere Entwicklung erleichtern.

14. Verfahren nach Anspruch 12(II), wobei das Genom des haploiden Embryos vor dem Schritt (II)(f) editiert wird, um eine vorteilhafte genetische Zusammensetzung zu erzeugen oder um Merkmale einzuführen, die das weitere Wachstum und die weitere Entwicklung erleichtern.

## Revendications

1. Procédé pour obtenir de manière non destructive le génotype d'un microspore, ledit procédé comprenant :
(a) l'isolation d'un tétrade de microspore comprenant quatre microspores tétradiques,
(b) la séparation du tétrade de microspore pour obtenir quatre microspores tétradiques,
(c) le génotypage de trois des quatre microspores tétradiques ; et
(d) l'inférence du génotype du quatrième microspore tétradique à partir des génotypes obtenus dans l'étape (c).

2. Procédé selon la revendication 1, dans lequel le tétrade de microspore provient de :
(i) maïs ; ou
(ii) canola.

3. Procédé selon la revendication 1, dans lequel le tétrade de microspore est mécaniquement séparé dans l'étape (b) pour obtenir quatre microspores tétradiques ; de préférence dans lequel :
(i) un micromanipulateur ou trieur de cellules ; ou
(ii) un système microfluidique est utilisé pour mécaniquement séparer le tétrade de microspore.

4. Procédé selon la revendication 1, dans lequel le tétrade de microspore est enzymatiquement séparé dans l'étape (b) pour obtenir quatre microspores tétradiques.

5. Procédé selon la revendication 1, comprenant en outre l'isolation du quatrième microspore tétradique après l'étape (b).

6. Procédé selon la revendication 1 :
(i) dans lequel avant le génotypage des trois microspores tétradiques, une amplification de génome entier est réalisée ; ou
(ii) dans lequel les trois microspores tétradiques sont génotypés individuellement ou qPCR est appliquée sur un échantillon groupé des trois microspores tétradiques.

7. Procédé d'obtention d'un microspore, comprenant :
(i) la réalisation des étapes (a) à (d) selon la revendication 1 ; et
(ii) la sélection du quatrième microspore tétradique sur la base de : un génotype préféré à au moins un locus, un génotype de génome entier, un génotype de tout le génome, au moins un chromosome à partir d'une espèce différente, un trait d'intérêt incluant, sans toutefois y être limité, des traits simples et complexes, une mutation, une invalidation génique, une délétion, une inactivation, un locus transgénique, un haplotype recombinant, un complément génétique à un autre génotype, ou une quelconque association de ceux-ci.

8. Procédé d'obtention d'un microspore doublé, comprenant :
(i) la réalisation des étapes (i) et (ii) selon la revendication 7 ; et
(ii) le placement du quatrième microspore tétradique sélectionné en contact avec un agent de doublage de chromosome ; de préférence dans lequel ledit agent de doublage de chromosome est colchicine.

9. Procédé de production d'un embryon haploïde doublé ou d'une plante haploïde doublée, comprenant :
(i) la réalisation des étapes (i) et (ii) selon la revendication 8 ; et
(ii) la production d'un embryon haploïde doublé à partir du microspore doublé ; optionnellement comprenant en outre :
(iii) la croissance de l'embryon haploïde doublé en une plante haploïde doublée.

10. Procédé de production d'un embryon haploïde à partir d'un microspore, comprenant :
(i) la réalisation des étapes (i) et (ii) selon la revendication 7 ; et
(ii) la production d'un embryon haploïde à partir du quatrième microspore tétradique sélectionné.

11. Procédé de production d'un embryon haploïde doublé, comprenant :
(i) la réalisation des étapes (i) et (ii) selon la revendication 10 ; et
(ii) le placement de l'embryon haploïde en contact avec un agent de doublage de chromosome ; de préférence dans lequel ledit agent de doublage de chromosome est colchicine.

12. Procédé pour créer une plante haploïde doublée, ledit procédé comprenant :
(a) l'isolation d'un tétrade de microspore ;
(b) la séparation du tétrade de microspore pour obtenir quatre microspores tétradiques ;
(c) le génotypage de trois des quatre microspores tétradiques ;
(d) l'inférence du génotype du quatrième microspore tétradique à partir des génotypes obtenu dans l'étape (c) ; et
(I)
(e) la mise en culture du quatrième microspore tétradique ;
(f) la mise en contact du quatrième microspore tétradique mis en culture avec un agent de doublage de chromosome ;
(g) la production d'un embryon haploïde doublé à partir du microspore doublé ; et
(h) la génération d'une plante haploïde doublée à partir de l'embryon haploïde doublé ; ou
(II)
(e) la production d'un embryon haploïde à partir du quatrième microspore tétradique ;
(f) la mise en contact de l'embryon haploïde avec un agent de doublage de chromosome ; et
(g) la croissance de l'embryon haploïde doublé en une plante haploïde doublée.

13. Procédé selon la revendication 12, dans lequel :
(a) ledit tétrade de microspore provient de :
(i) maïs ; ou
(ii) canola ;
(b) ledit agent de doublage de chromosome est colchicine ; ou
(c) le génome du quatrième microspore tétradique est édité avant l'étape (I)(f) pour générer une composition génétique favorable ou pour introduire des traits qui facilitent une croissance et un développement supplémentaires.

14. Procédé selon la revendication 12(II), dans lequel le génome de l'embryon haploïde est édité avant l'étape (II)(f) pour générer une composition génétique favorable ou pour introduire des traits qui facilitent une croissance et un développement supplémentaires.
